# EUROPEAN PATENT APPLICATION

(11) **EP 2 248 520 A2**
(43) Date of publication of application: **10.11.2010**
(21) Application number: 10008427.6
(22) Date of filing: 06.10.2005
(51) Int. Cl.: A61K 31/00, A61K 31/165, A61K 31/451, A61P 9/00

(54) **Use of renin inhibitors for the prevention or treatment of diastolic dysfunction or diastolic heart failure**

(30) Priority: 08.10.2004 US 617202 P; 01.04.2005 US 667899 P
(62) Divisional of application: 05810560.2
(71) Applicant: Novartis AG, 4056 Basel (CH); Novartis Pharma GmbH, 1230 Wien (AT)
(72) Inventor: Feldman, David Louis, Teaneck, New Jersey 07666 (US); Luft, Friedrich Cameron, 16341 Panketal (DE); Mueller, Dominik Nicolas, 10405 Berlin (DE); Webb, Randy lee, Flemington, New Jersey 08822 (US)
(74) Representative: Grünecker, Kinkeldey, Stockmair & Schwanhäusser Anwaltssozietät

(57) **Abstract**

A pharmaceutical composition comprising a renin inhibitor for the prevention of, delay progression to overt to, or the treatment of diastolic dysfunction or diastolic heart failure.

## Description

The natural enzyme renin passes from the kidneys into the blood where it effects the cleavage of angiotensinogen, releasing the decapeptide angiotensin I which is then cleaved in the lungs, the kidneys and other organs to form the octapeptide angiotensinogen II. The octapeptide increases blood pressure both directly by arterial vasoconstriction and indirectly by liberating from the adrenal glands the sodium-ion-retaining hormone aldosterone, accompanied by an increase in extracellular fluid volume. That increase can be attributed to the action of angiotensin II. Inhibitors of the enzymatic activity of renin bring about a reduction in the formation of angiotensin I. As a result a smaller amount of angiotensin II is produced. The reduced concentration of that active peptide hormone is the direct cause of, e.g., the hypotensive effect of renin inhibitors.

Further evaluations have revealed that renin inhibitors may also be employed for a broader range of therapeutic indications.

It has now surprisingly been found that renin inhibitors may be employed for the treatment of diastolic dysfunction and diastolic heart failure by controlling blood pressure and volume. Even more surprisingly, renin inhibitors have been found to delay the onset of or even to reverse the progression of left ventricular (LV) hypertrophy and its attendant increase in cardiac fibrosis by suppressing the levels of the profibrogenic angiotensin II.

Accordingly, the present invention relates to a method for the prevention of, delay progression to overt to, or the treatment of diastolic dysfunction or diastolic heart failure which method comprises administering to a warm-blooded animal a therapeutically effective amount of a renin inhibitor, or a pharmaceutically acceptable salt thereof.

Diastolic dysfunction as used herein refers to abnormal mechanical properties of the heart muscle (myocardium) and includes abnormal LV diastolic distensibility, impaired filling, and slow or delayed relaxation regardless of whether the ejection fraction is normal or depressed and whether the patient is asymptomatic or symptomatic. Asymptomatic diastolic dysfunction is used to refer to an asymptomatic patient with a normal ejection fraction and an abnormal echo-Doppler pattern of LV filling which is often seen, for example, in patients with hypertensive heart disease.

Thus, an asymptomatic patient with hypertensive left ventricular hypertrophy and an echocardiogram showing a normal ejection fraction and abnormal left ventricular filling can be said to have diastolic dysfunction.

If such a patient were to exhibit symptoms of effort intolerance and dyspnea, especially if there were evidence of venous congestion and pulmonary edema, it would be more appropriate to use the term diastolic heart failure. This terminology parallels that used in asymptomatic and symptomatic patients with LV systolic dysfunction, and it facilitates the use of a pathophysiologic, diagnostic, and therapeutic framework that includes all patients with LV dysfunction whether or not they have symptoms (William H. Gaasch and Michael R. Zile, Annu. Rev. Med. 2004, 55:373-94; Gerard P. Aurigemma, William H. Gaasch, N. Engl. J. Med. 2004, 351:1097-105).

In other words, in order for the heart to pump effectively the LV must be able to accept blood (coming from the left atrium) into its chamber for subsequent pumping to the aorta. Accommodating the blood from the left atrium is dependent in part on how much the LV can relax and distend in response to the inflow of blood. Sometimes the LV can not distend enough to accommodate the volume of blood from the left atrium, resulting in impaired filling (with blood) of the LV. This can happen due to mechanical dysfunction of the myocardium. It leads to abnormal (low) ejection fraction, i.e., fraction of blood in LV that is actually pumped out.

Among the factors that lead to diastolic dysfunction or diastolic heart failure, uncontrolled hypertension and fluid retention are prominent. Renin inhibitors are known to lower blood pressure at least as effectively as angiotensin converting enzyme (ACE) inhibitors and angiotensin II receptor blockers (ARBs, also called AT₁-receptor antagonists), thus suggesting a delay in onset of the development of diastolic dysfunction due to their antihypertensive effect. Furthermore, since renin inhibitors effectively modulate the generation of antiotensin II, aldosterone levels are also expected to be lowered and, therefore, renin inhibitors may also limit fluid retention. On the basis of the anti-fibrotic properties of blockers of the renin angiotensin system (RAS), especially renin inhibitors, such agents may inhibit the development of LV hypertrophy and its attendant increase in cardiac fibrosis by suppressing the levels of the profibrogenic angiotensin II.

Furthermore, it has now been shown that a combination of a renin inhibitor with an (i) ACE inhibitor or (ii) an angiotensin II receptor blocker confers added or synergistic therapeutic effects over each monotherapy component alone.

Accordingly, the present invention further relates to a method for the prevention of, delay progression to overt to, or the treatment of diastolic dysfunction or diastolic heart failure which method comprises administering to a warm-blooded animal a therapeutically effective amount of a combination of a renin inhibitor, or a pharmaceutically acceptable salt thereof, with
(i) an ACE inhibitor, or a pharmaceutically acceptable salt thereof; or
(II) an angiotensin II receptor blocker, or a pharmaceutically acceptable salt thereof.

Other objects, features, advantages and aspects of the present invention will become apparent to those skilled in the art from the following description and appended claims. It should be understood, however, that the following description, appended claims, and specific examples, while indicating preferred embodiments of the invention, are given by way of illustration only. Various changes and modifications within the spirit and scope of the disclosed invention will become readily apparent to those skilled in the art from reading the following. Abbreviations are those generally known in the art.

Listed below are the definitions of various terms used herein to describe certain aspects of the present invention. However, the definitions and abbreviations thereof used herein are those generally known in the art and apply to the terms as they are used throughout the specification unless they are otherwise limited in specific instances.

The term "prevention" refers to prophylactic administration to healthy patients to prevent the development of the conditions mentioned herein. Moreover, the term "prevention" means prophylactic administration to patients being in a pre-stage of the conditions to be treated.

The term "delay progression to overt to", as used herein, refers to administration to patients being in a pre-stage of the condition to be treated in which patients with a pre-form of the corresponding condition is diagnosed.

The term "treatment" is understood the management and care of a patient for the purpose of combating the disease, condition or disorder.

The term "therapeutically effective amount" refers to an amount of a drug or a therapeutic agent that will elicit the desired biological or medical response of a tissue, system or an animal (including man) that is being sought by a researcher or clinician.

The term "synergistic", as used herein, means that the effect achieved with the methods, combinations and pharmaceutical compositions of the present invention is greater than the sum of the effects that result from individual methods and compositions comprising the active ingredients of this invention separately.

The term "warm-blooded animal or patient" are used interchangeably herein and include, but are not limited to, humans, dogs, cats, horses, pigs, cows, monkeys, rabbits, mice and laboratory animals. The preferred mammals are humans.

The term "pharmaceutically acceptable salt" refers to a non-toxic salt commonly used in the pharmaceutical industry which may be prepared according to methods well-known in the art.

The term "combination" of a renin inhibitor, in particular, aliskiren, and an ACE inhibitor or an angiotensin II receptor blocker, or in each case, a pharmaceutically acceptable salt thereof, means that the components can be administered together as a pharmaceutical composition or as part of the same, unitary dosage form. A combination also includes administering a renin inhibitor, in particular, aliskiren, or a pharmaceutically acceptable salt thereof, and an ACE inhibitor or an angiotensin II receptor blocker, or in each case, a pharmaceutically acceptable salt thereof, each separately but as part of the same therapeutic regimen. The components, if administered separately, need not necessarily be administered at essentially the same time, although they can if so desired. Thus, a combination also refers, for example, administering a renin inhibitor, in particular, aliskiren, or a pharmaceutically acceptable salt thereof, and an ACE inhibitor or an angiotensin II receptor blocker, or in each case, a pharmaceutically acceptable salt thereof, as separate dosages or dosage forms, but at the same time. A combination also includes separate administration at different times and in any order.

The renin inhibitors to which the present invention applies are any of those having renin inhibitory activity *in vivo* and, therefore, pharmaceutical utility, e.g., as therapeutic agents for the prevention of, delay progression to overt to, or the treatment of diastolic dysfunction or diastolic heart failure. In particular, the present invention relates to renin inhibitors disclosed in U.S. Patents No. 5,559,111; No. 6,197,959 and No. 6,376,672, the entire contents of which are incorporated herein by reference.

Renin inhibitors include compounds having different structural features. For example, mention may be made of compounds which are selected from the group consisting of ditekiren (chemical name: [1S-[1R*,2R*,4R*(1R*,2R*)]]-1-[(1,1-dimethylethoxy)carbonyl]-L-proly I-L-phenylalanyl-N-[2-hydroxy-5-methyl-1-(2-methylpropyl)-4-[[[2-methyl-1-[[(2-pyridinylmrthyl)amino]carbonyl]butyl]amino]carbonyl]hexyl]-N-alfa-methyl-L-histidinamide); terlakiren (chemical name: [R-(R*,S*)]-N-(4-morpholinylcarbonyl)-L-phenylalanyl-N-[1-(cyclohexy lmethyl)-2-hydroxy-3-(1-methylethoxy)-3-oxopropyl]-S-methyl-L-cysteineamide); and zankiren (chemical name: [1S-[1R*[R*(R*)],2S*,3R*]]-N-[1-(cyclohexylmethyl)-2,3-dihydroxy-5-methylhexyl]-alfa-[[2-[[(4-methyl-1-piperazinyl)sulfonyl]methyl]-1-oxo-3-phenylpropyl]-amino]-4-thiazolepropanamide), preferably, in each case, the hydrochloride salt thereof.

Preferred renin inhibitor of the present invention include RO 66-1132 and RO 66-1168 of formulae (I) and (II) 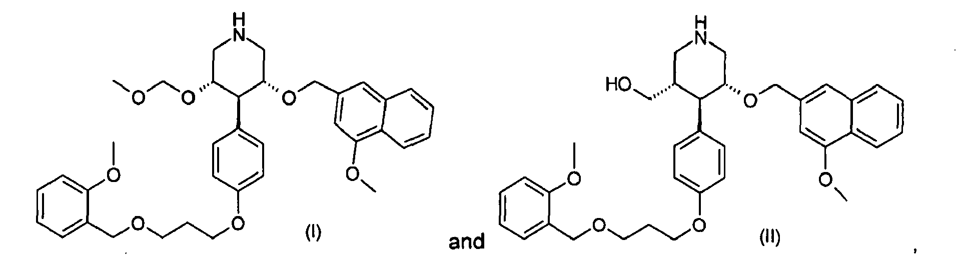 respectively, or in each case, a pharmaceutically acceptable salt thereof.

In particular, the present invention relates to a renin inhibitor which is is a δ-amino-γ-hydroxy-ω-aryl-alkanoic acid amide derivative of the formula 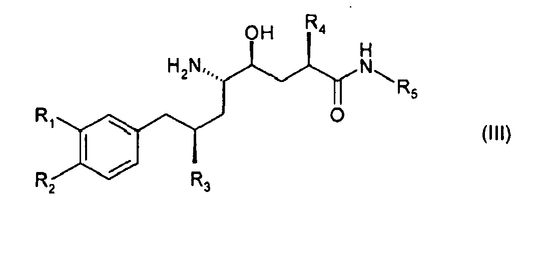 wherein R₁ is halogen, C₁₋₆halogenalkyl, C₁₋₆alkoxy-C₁₋₆alkyloxy or C₁₋₆alkoxy-C₁₋₆alkyl; R₂ is halogen, C₁₋₄alkyl or C₁₋₄alkoxy; R₃ and R₄ are independently branched C₃₋₆alkyl; and R₅ is cycloalkyl, C₁₋₆alkyl, C₁₋₆hydroxyalkyl, C₁₋₆alkoxy-C₁₋₆alkyl, C₁₋₆alkanoyloxy-C₁₋₆alkyl, C₁₋₆aminoalkyl, C₁₋₆alkylamino-C₁₋₆alkyl, C₁₋₆dialkylamino-C₁₋₆alkyl, C₁₋₆alkanoylamino-C₁₋₆alkyl, HO(O)C-C₁₋₆alkyl, C₁₋₆alkyl-O-(O)C-C₁₋₆alkyl, H₂N-C(O)-C₁₋₆alkyl, C₁₋₆alkyl-HN-C(O)-C₁₋₆alkyl or (C₁₋₆alkyl)₂N-C(O)-C₁₋₆alkyl; or a pharmaceutically acceptable salt thereof.

As an alkyl, R₁ may be linear or branched and preferably comprise 1 to 6 C atoms, especially 1 or 4 C atoms. Examples are methyl, ethyl, n- and i-propyl, n-, i- and t-butyl, pentyl and hexyl.

As a halogenalkyl, R₁ may be linear or branched and preferably comprise 1 to 4 C atoms, especially 1 or 2 C atoms. Examples are fluoromethyl, difluoromethyl, trifluoromethyl, chloromethyl, dichloromethyl, trichloromethyl, 2-chloroethyl and 2,2,2-trifluoroethyl.

As an alkoxy, R₁ and R₂ may be linear or branched and preferably comprise 1 to 4 C atoms. Examples are methoxy, ethoxy, n- and i-propyloxy, n-, i- and t-butyloxy, pentyloxy and hexyloxy.

As an alkoxyalkyl, R₁ may be linear or branched. The alkoxy group preferably comprises 1 to 4 and especially 1 or 2 C atoms, and the alkyl group preferably comprises 1 to 4 C atoms. Examples are methoxymethyl, 2-methoxyethyl, 3-methoxypropyl, 4-methoxybutyl, 5-methoxypentyl, 6-methoxyhexyl, ethoxymethyl, 2ethoxyethyl, 3-ethoxypropyl, 4-ethoxybutyl, 5-ethoxypentyl, 6-ethoxyhexyl, propyloxymethyl, butyloxymethyl, 2-propyloxyethyl and 2-butyloxyethyl.

As a C₁₋₆alkoxy-C₁₋₆alkyloxy, R₁ may be linear or branched. The alkoxy group preferably comprises 1 to 4 and especially 1 or 2 C atoms, and the alkyloxy group preferably comprises 1 to 4 C atoms. Examples are methoxymethyloxy, 2-methoxyethyloxy, 3-methoxypropyloxy, 4-methoxybutyloxy, 5-methoxypentyloxy, 6-methoxyhexyloxy, ethoxymethyloxy, 2-ethoxyethyloxy, 3-ethoxypropyloxy, 4-ethoxybutyloxy, 5-ethoxypentyloxy, 6-ethoxyhexyloxy, propyloxymethyloxy, butyloxymethyloxy, 2-propyloxyethyloxy and 2-butyloxyethyloxy.

In a preferred embodiment, R₁ is methoxy- or ethoxy-C₁₋₄alkyloxy, and R₂ is preferably methoxy or ethoxy. Particularly preferred are compounds of formula (III), wherein R₁ is 3-methoxypropyloxy and R₂ is methoxy.

As a branched alkyl, R₃ and R₄ preferably comprise 3 to 6 C atoms. Examples are i-propyl, i- and t-butyl, and branched isomers of pentyl and hexyl. In a preferred embodiment, R₃ and R₄ in compounds of formula (III) are in each case i-propyl.

As a cycloalkyl, R₅ may preferably comprise 3 to 8 ring-carbon atoms, 3 or 5 being especially preferred. Some examples are cyclopropyl, cyclobutyl, cyclopentyl, cyclohexyl and cyclooctyl. The cycloalkyl may optionally be substituted by one or more substituents, such as alkyl, halo, oxo, hydroxy, alkoxy, amino, alkylamino, dialkylamino, thiol, alkylthio, nitro, cyano, heterocyclyl and the like.

As an alkyl, R₅ may be linear or branched in the form of alkyl and preferably comprise 1 to 6 C atoms. Examples of alkyl are listed herein above. Methyl, ethyl, n- and i-propyl, n-, i- and t-butyl are preferred.

As a C₁₋₆hydroxyalkyl, R₅ may be linear or branched and preferably comprise 2 to 6 C atoms. Some examples are 2-hydroxyethyl, 2-hydroxypropyl, 3-hydroxypropyl, 2-, 3- or 4-hydroxybutyl, hydroxypentyl and hydroxyhexyl.

As a C₁₋₆alkoxy-C₁₋₆alkyl, R₅ may be linear or branched. The alkoxy group preferably comprises 1 to 4 C atoms and the alkyl group preferably 2 to 4 C atoms. Some examples are 2-methoxyethyl, 2-methoxypropyl, 3-methoxypropyl, 2-, 3- or 4-methoxybutyl, 2-ethoxyethyl, 2-ethoxypropyl, 3-ethoxypropyl, and 2-, 3- or 4-ethoxybutyl.

As a C₁₋₆alkanoyloxy-C₁₋₆alkyl, R₅ may be linear or branched. The alkanoyloxy group preferably comprises 1 to 4 C atoms and the alkyl group preferably 2 to 4 C atoms. Some examples are formyloxymethyl, formyloxyethyl, acetyloxyethyl, propionyloxyethyl and butyroyloxyethyl.

As a C₁₋₆aminoalkyl, R₅ may be linear or branched and preferably comprise 2 to 4 C atoms. Some examples are 2-aminoethyl, 2- or 3-aminopropyl and 2-, 3- or 4-aminobutyl.

As C₁₋₆alkylamino-C₁₋₆alkyl and C₁₋₆dialkylamino-C₁₋₆alkyl, R₅ may be linear or branched. The alkylamino group preferably comprises C₁₋₄alkyl groups and the alkyl group has preferably 2 to 4 C atoms. Some examples are 2-methylaminoethyl, 2-dimethylaminoethyl, 2-ethylaminoethyl, 2-ethylaminoethyl, 3-methylaminopropyl, 3-dimethylaminopropyl, 4-methylaminobutyl and 4-dimethylaminobutyl.

As a HO(O)C-C₁₋₆alkyl, R₅ may be linear or branched and the alkyl group preferably comprises 2 to 4 C atoms. Some examples are carboxymethyl, carboxyethyl, carboxypropyl and carboxybutyl.

As a C₁₋₆alkyl-O-(O)C-C₁₋₆alkyl, R₅ may be linear or branched, and the alkyl groups preferably comprise independently of one another 1 to 4 C atoms. Some examples are methoxycarbonylmethyl, 2-methoxycarbonylethyl, 3-methoxycarbonylpropyl, 4-methoxy-carbonylbutyl, ethoxycarbonylmethyl, 2-ethoxycarbonylethyl, 3-ethoxycarbonylpropyl, and 4-ethoxycarbonylbutyl.

As a H₂N-C(O)-C₁₋₆alkyl, R₅ may be linear or branched, and the alkyl group preferably comprises 2 to 6 C atoms. Some examples are carbamidomethyl, 2-carbamidoethyl, 2-carbamido-2,2-dimethylethyl, 2- or 3-carbamidopropyl, 2-, 3- or 4-carbamidobutyl, 3-carbamido-2-methylpropyl, 3-carbamido-1,2-dimethylpropyl, 3-carbamido-3-ethylpropyl, 3-carbamido-2,2-dimethylpropyl, 2-, 3-, 4- or 5-carbamidopentyl, 4-carbamido-3,3- or -2,2-dimethylbutyl.

Accordingly, preferred are δ-amino-γ-hydroxy-ω-aryl-alkanoic acid amide derivatives of formula (III) having the formula 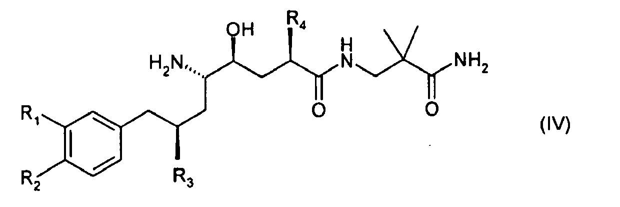 wherein R₁ is 3-methoxypropyloxy; R₂ is methoxy; and R₃ and R₄ are isopropyl; or a pharmaceutically acceptable salt thereof; chemically defined as 2(S),4(S),5(S),7(S)-N-(3-amino-2,2-dimethyl-3-oxopropyl)-2,7-di(1-methylethyl)-4-hydroxy-5-amino-8-[4-methoxy-3-(3-methoxy-propoxy)phenyl]-octanamide, also known as aliskiren.

The term "aliskiren", if not defined specifically, is to be understood both as the free base and as a salt thereof, especially a pharmaceutically acceptable salt thereof, most preferably a hemi-fumarate thereof.

Angiotensin II receptor blockers are understood to be those active agents that bind to the AT₁-receptor subtype of angiotensin II receptor but do not result in activation of the receptor. As a consequence of the blockade of the AT₁ receptor, these antagonists can, e.g., be employed as antihypertensive agents.

Suitable angiotensin II receptor blockers which may be employed in the combination of the present invention include AT₁ receptor antagonists having differing structural features, preferred are those with the non-peptidic structures. For example, mention may be made of the compounds that are selected from the group consisting of valsartan (EP 443983), losartan (EP253310), candesartan (EP 459136), eprosartan (EP 403159), irbesartan (EP 454511), olmesartan (EP 503785), tasosartan (EP539086), telmisartan (EP 522314), the compound with the designation E-4177 of the formula 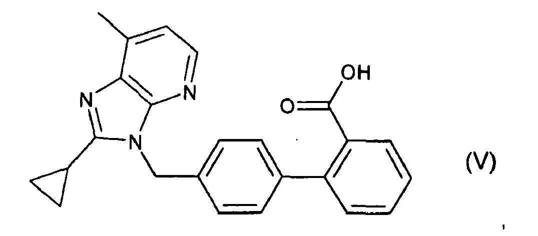 the compound with the designation SC-52458 of the following formula 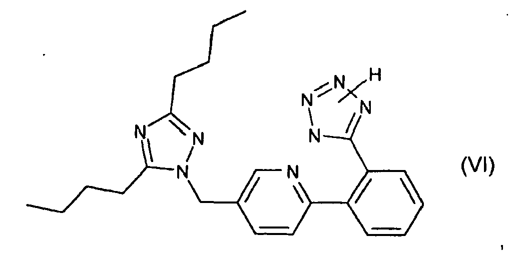 and the compound with the designation the compound ZD-8731 of the formula 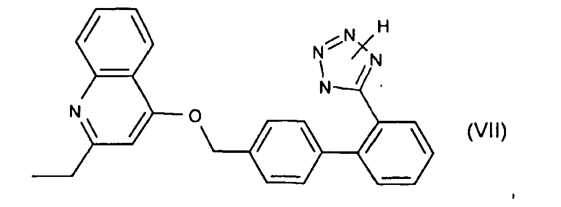 or, in each case, a pharmaceutically acceptable salt thereof.

Preferred AT₁-receptor antagonists are those agents that have reach the market, most preferred is valsartan, or a pharmaceutically acceptable salt thereof.

The interruption of the enzymatic degradation of angiotensin I to angiotensin II with ACE inhibitors is a successful variant for the regulation of blood pressure and thus also makes available a therapeutic method for the treatment of hypertension.

A suitable ACE inhibitor to be employed in the combination of the present invention is, e.g., a compound selected from the group consisting alacepril, benazepril, benazeprilat, captopril, ceronapril, cilazapril, delapril, enalapril, enaprilat, fosinopril, imidapril, lisinopril, moveltopril, perindopril, quinapril, ramipril, spirapril, temocapril, and trandolapril, or in each case, a pharmaceutically acceptable salt thereof.

Preferred ACE inhibitors are those agents that have been marketed, most preferred are benazepril and enalapril.

Preferably, a combination according to the present invention comprises a renin inhibitor, e.g., aliskiren, especially in the form of the hemi-fumarate salt thereof, and an ACE inhibitor, e.g., benazepril or enalapril, or an angiotensin II receptor blocker, e.g., valsartan, or in each case, a pharmaceutically acceptable salt thereof.

Most preferred is a combination according to the present invention comprising aliskiren, especially in the form of the hemi-fumarate salt thereof, and valsartan, or a pharmaceutically acceptable salt thereof.

As referred herein above, the compounds to be combined may be present as their pharmaceutically acceptable salts. If these compounds have, e.g., at least one basic center such as an amino group, they can form acid addition salts thereof. Similarly, the compounds having at least one acid group (for example COOH) can form salts with bases.

Corresponding internal salts may furthermore be formed, if a compound comprises, e.g., both a carboxy and an amino group.

The corresponding active ingredients or a pharmaceutically acceptable salts may also be used in form of a solvate, such as a hydrate or including other solvents used, e.g., in their crystallization.

Furthermore, the present invention provides pharmaceutical compositions comprising a renin inhibitor, or a pharmaceutically acceptable salt thereof, preferably aliskiren in the form of the hemi-fumarate salt thereof, and a pharmaceutically acceptable carrier, for the prevention of, delay progression to overt to, or the treatment of diastolic dysfunction or diastolic heart failure.

In another aspect, the present invention further provides pharmaceutical compositions comprising a renin inhibitor, or a pharmaceutically acceptable salt thereof, preferably aliskiren in the form of the hemi-fumarate salt thereof, in combination with
(i) an ACE inhibitor, preferably benazepril or enalapril, or in each case, a pharmaceutical acceptable salt thereof; or
(ii) an angiotensin II receptor blocker, preferably valsartan, or a pharmaceutically acceptable salt thereof;
and a pharmaceutically acceptable carrier; for the prevention of, delay progression to overt to, or the treatment of diastolic dysfunction or diastolic heart failure.

As disclosed herein above, a renin inhibitor, in particular, aliskiren, preferably in the form of the hemi-fumarate salt thereof, alone or in combination with an ACE inhibitor, e.g., benazepril or enalapril, or an angiotensin II receptor blocker, e.g., valsartan, or in each case, a pharmaceutically acceptable salt thereof, may be co-administered as a pharmaceutical composition. The components may be administered together in any conventional dosage form, usually also together with a pharmaceutically acceptable carrier or diluent.

The pharmaceutical compositions according to the invention are those suitable for enteral, such as oral or rectal, transdermal and parenteral administration to mammals, including man. For oral administration the pharmaceutical composition comprising a renin inhibitor, in particular, aliskiren, preferably in the form of the hemi-fumarate salt thereof, alone or in combination with an ACE inhibitor, e.g., benazepril or enalapril, or an angiotensin II receptor blocker, e.g., valsartan, or in each case, a pharmaceutically acceptable salt thereof, can take the form of solutions, suspensions, tablets, pills, capsules, powders, microemulsions, unit dose packets and the like. Preferred are tablets and gelatin capsules comprising the active ingredient together with: a) diluents, e.g., lactose, dextrose, sucrose, mannitol, sorbitol, cellulose and/or glycine; b) lubricants, e.g., silica, talcum, stearic acid, its magnesium or calcium salt and/or polyethyleneglycol; for tablets also c) binders, e.g., magnesium aluminum silicate, starch paste, gelatin, tragacanth, methylcellulose, sodium carboxymethylcellulose and or polyvinylpyrrolidone; if desired d) disintegrants, e.g., starches, agar, alginic acid or its sodium salt, or effervescent mixtures; and/or e) absorbants, colorants, flavors and sweeteners. Injectable compositions are preferably aqueous isotonic solutions or suspensions, and suppositories are advantageously prepared from fatty emulsions or suspensions.

Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1-90%, preferably about 1-80%, of the active ingredient.

The dosage of the active ingredients can depend on a variety of factors, such as mode of administration, homeothermic species, age and/or individual condition.

Preferred dosages for the active ingredients of the pharmaceutical combinations according to the present invention are therapeutically effective dosages, especially those which are commercially available.

Normally, in the case of oral administration, an approximate daily dose of from about 1 mg to about 360 mg is to be estimated, e.g., for a patient of approximately 75 kg in weight.

For example, the doses of aliskiren to be administered to warm-blooded animals, including man, of approximately 75 kg body weight, especially the doses effective for the inhibition of renin activity, e.g., in lowering blood pressure, are from about 3 mg to about 3 g, preferably from about 10 mg to about 1 g, e.g., from 20 to 200 mg/person/day, divided preferably into 1 to 4 single doses which may, e.g., be of the same size. Usually, children receive about half of the adult dose. The dose necessary for each individual can be monitored, e.g., by measuring the serum concentration of the active ingredient, and adjusted to an optimum level. Single doses comprise, e.g., 75 mg, 150 mg or 300 mg per adult patient.

In case of ACE inhibitors, preferred dosage unit forms of ACE inhibitors are, for example, tablets or capsules comprising e.g. from about 5 mg to about 20 mg, preferably 5 mg, 10 mg, 20 mg or 40 mg, of benazepril; from about 6.5 mg to 100 mg, preferably 6.25 mg, 12.5 mg, 25 mg, 50 mg, 75 mg or 100 mg, of captopril; from about 2.5 mg to about 20 mg, preferably 2.5 mg, 5 mg, 10 mg or 20 mg, of enalapril; from about 10 mg to about 20 mg, preferably 10 mg or 20 mg, of fosinopril; from about 2.5 mg to about 4 mg, preferably 2 mg or 4 mg, of perindopril; from about 5 mg to about 20 mg, preferably 5 mg, 10 mg or 20 mg, of quinapril; or from about 1.25 mg to about 5 mg, preferably 1.25 mg, 2.5 mg, or 5 mg, of ramipril. Preferred is t.i.d. administration.

Angiotensin II receptor blockers, e.g., valsartan, are supplied in the form of a suitable dosage unit form, e.g., a capsule or tablet, and comprising a therapeutically effective amount of an angiotensin II receptor blocker, e.g., from about 20 to about 320 mg of valsartan, which may be applied to patients. The application of the active ingredient may occur up to three times a day, starting, e.g., with a daily dose of 20 mg or 40 mg of an angiotensin II receptor blocker, e.g., valsartan, increasing via 80 mg daily and further to 160 mg daily, and finally up to 320 mg daily. Preferably, an angiotensin II receptor blocker, e.g., valsartan is applied once a day or twice a day with a dose of 80 mg or 160 mg, respectively, each. Corresponding doses may be taken, e.g., in the morning, at mid-day or in the evening.

The above doses encompass a therapeutically effective amount of the active ingredients of the present invention.

Since the present invention relates to methods for the prevention, delay progression to overt to, or the treatment with a combination of compounds which may be administered separately, the invention also relates to combining separate pharmaceutical compositions in a kit form. The kit may comprise, e.g., two separate pharmaceutical compositions: (1) a composition comprising a renin inhibitor, in particular, aliskiren, or a pharmaceutically acceptable salt thereof, and a pharmaceutically acceptable carrier or diluent; and (2) a composition comprising an ACE inhibitor, e.g., benazepril or enalapril, or an angiotensin II receptor blocker, e.g., valsartan, or in each case, a pharmaceutically acceptable salt thereof, and a pharmaceutically acceptable carrier or diluent. The amounts of (1) and (2) are such that, when co-administered separately a beneficial therapeutic effect(s) is achieved. The kit comprises a container for containing the separate compositions such as a divided bottle or a divided foil packet, wherein each compartment contains a plurality of dosage forms (e.g., tablets) comprising, e.g., (1) or (2). Alternatively, rather than separating the active ingredient-containing dosage forms, the kit may contain separate compartments each of which contains a whole dosage which in turn comprises separate dosage forms. An example of this type of kit is a blister pack wherein each individual blister contains two (or more) tablets, one (or more) tablet(s) comprising a pharmaceutical composition (1), and the second (or more) tablet(s) comprising a pharmaceutical composition (2). Typically the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician. In the case of the instant invention a kit therefore comprises:
(1) a therapeutically effective amount of a composition comprising a renin inhibitor, in particular, aliskiren, preferably in the form of the hemi-fumarate salt thereof, and a pharmaceutically acceptable carrier or diluent, in a first dosage form;
(2) a composition comprising an ACE inhibitor, e.g., benazepril or enalapril, or an angiotensin II receptor blocker, e.g., valsartan, or in each case, a pharmaceutically acceptable salt thereof, in an amount such that, following administration, a beneficial therapeutic effect(s) is achieved, and a pharmaceutically acceptable carrier or diluent, in a second dosage form; and
(3) a container for containing said first and second dosage forms.

The action of a renin inhibitor, e.g., aliskiren, may be demonstrated *inter alia* experimentally by means of *in vitro* tests, the reduction in the formation of angiotensin I being measured in various systems (human plasma, purified human renin together with synthetic or natural renin substrate).

Since renin displays species specificity for its substrate, human renin inhibitors cannot be efficiently tested in conventional *in vivo* animal models. To circumvent this problem, transgenic rats have been developed harboring either the human renin or the human angiotensinogen genes. Human renin does not effectively cleave rat angiotensinogen and similarly, rat renin cleaves human angiotensinogen poorly. Consequently, the single transgenic rats (i.e., transgenic for either human angiotensinogen or renin) are normotensive. However, when crossbred, the double transgenic (dTGR) offspring develop, e.g., hypertension and diastolic dysfunction, and do not live beyond the 7^{th} or 8^{th} week of age.

A renin inhibitor, e.g., aliskiren, or a pharmaceutically acceptable salt thereof, alone or in combination with an ACE inhibitor, e.g., benazepril or enalapril, or an angiotensin II receptor blocker, e.g., valsartan, or in each case, a pharmaceutically acceptable salt thereof, can be administered by various routes of administration. Each agent can be tested over a wide-range of dosages to determine the optimal drug level for each therapeutic agent alone, or in the specific combination thereof, to elicit the maximal response. For these studies, it is preferred to use treatment groups consisting of at least 6 animals per group. Each study is best performed in away wherein the effects of the combination treatment group are determined at the same time as the individual components are evaluated. Although drug effects may be observed with acute administration, it is preferable to observe responses in a chronic setting. The long-term study is of sufficient duration to allow for the full development of compensatory responses to occur and, therefore, the observed effect will most likely depict the actual responses of the test system representing sustained or persistent effects.

Accordingly, a renin inhibitor, or a pharmaceutically acceptable salt thereof, alone or in combination with an ACE inhibitor or an angiotensin II receptor blocker, or in each case, a pharmaceutically acceptable salt thereof, can be tested for its inhibitory effects on diastolic dysfunction or diastolic heart failure in the double transgenic rats expressing human renin and human angiotensinogen (dTGR). For example, animals may be treated with aliskiren (1 mg/kg/day - 30 mg/kg/day) before the development of diastolic dysfunction (prevention design) or after developing diastolic dysfunction (treatment design). Measurements for cardiac function can be made with Tissue-Doppler imaging of rat hearts *in vivo.*

Similarly, a renin inhibitor, or a pharmaceutically acceptable salt thereof, alone or in combination with an ACE inhibitor or an angiotensin II receptor blocker, or in each case, a pharmaceutically acceptable salt thereof, may be tested for its inhibitory effects on diastolic dysfunction or diastolic heart failure in Ren-2 transgenic rats, expressing the mouse ren-2 (renin) gene. These rats can be made diabetic by injection with streptozotocin and diastolic dysfunction can be induced by ligating (tying off) a coronary artery to induce a myocardial infarction. Over the ensuing ∼1 month cardiac fibrosis and diastolic dysfunction develop. For example, animals may be treated with aliskiren (1 mg/kg/day - 60 mg/kg/day) before the development of diastolic dysfunction (prevention design) or after developing diastolic dysfunction (treatment design). Measurements for cardiac function can be made with Tissue-Doppler imaging of rat hearts *in vivo.*

As an example, four-week-old, male dTGR are allowed to develop hypertension and are placed in metabolic cages at 5.5 weeks of age. Systolic blood pressure (tail-cuff) and 24 h albumin excretion (ELISA, CellTrend, Germany) are measured as described earlier by Muller et al. Am J Pathol. 2002, 161:1679-93 and Muller et al. Am J Pathol. 2004, 164:521-32. The dTGR are matched at week 6 in terms of 24 h albumin excretion and distributed in five groups of 19 rats each. Treatments begin when the rats are aged 6 weeks. The rats receive vehicle treatment, aliskiren at 0.3 mg/kg/day and 3 mg/kg/day (by subcutaneous minipump), valsartan 1 mg/kg/day, and valsartan 10 mg/kg/day (given in the food). The low dose of valsartan is selected as a threshold treatment to reduce mortality yet only minimally effect blood pressure and organ damage. We know from earlier studies that vehicle-treated animals would not survive beyond 8 weeks of age and thus this low dose valsartan group will serve as a control group at 9 weeks. Echocardiography (M-mode tracings in the short axis and Tissue-Doppler-imaging; n=5-6 per group at weeks 7 and 9) is performed with a 15 MHz phased-array transducer under isoflurane anesthesia (Mazak et al. Circulation. 2004; 109:2792-800). Three measurements per heart are determined, averaged, and statistically analyzed. M-mode is performed in a LV short axis and measured according to the leading edge-method. Total wall thickness is calculated as sum of septum+left ventricular posterior wall.

Tissue Doppler measures the velocity of the longitudinal cardiac movement at the basal septum, allowing assessment of diastolic filling Tissue Doppler measurements are performed with the sample volume in the basal septum in a four-chamber view. Velocity range, gain, and filter settings are optimized to detect low velocities and the pulsed-wave Doppler spectrum is displayed at 200 mm/s. The measurements represent velocities of peak early (Ea) and late (Aa) diastolic expansion velocities. The Ea/Aa ratio is reported as an index of diastolic function.

Rats are sacrificed at age 9 weeks. The kidneys and hearts are removed and washed with ice-cold saline, blotted dry, and weighed. Tissue preparation and immunohistological techniques are performed as previously described Muller et al. Am J Pathol. 2002, 161:1679-93. Sections are incubated with primary antibodies against rat monocytes/macrophages (ED-1, Serotec, Germany), MHC II+, CD4+, and CD86+ cells (all BD Pharmingen, Germany). Scoring of infiltrated cells is performed using the program KS 300 3.0 (Zeiss, Germany). Fifteen different areas of each kidney (n=5 in all groups) are analyzed. A mean score for each animal is computed and used to derive a group mean score. Analyses are conducted without knowledge of the specific treatment.

For RT-PCR, LV mRNA is isolated with TRIZOL (Gibco Life Technology). RT-PCR for α-myosin heavy chain (α-MHC) and β-MHC, as well as for atrial natriuretic peptide (ANP) is carried out in 25 µL SybrGreen PCR Master Mix (Applied Biosystems, Germany) containing 0.3 or 0.9 mol/L primer and 1 µL of the reverse transcription reaction in a 5700 Sequence Detection System (Applied Biosystems). Thermal cycling conditions comprise an initial denaturation step at 95°C for 10 min, followed by 95°C for 15 s and 65°C for 1 min for 40 cycles. mRNA expression is standardized to the hypoxanthine phosphoribosyl transferase gene as a housekeeping gene (primer sequences available on request).

At sacrifice, the cardiac hypertrophy index score decreases in the valsartan 10 mg/kg/d, aliskiren 0.3 mg/kg/d and aliskiren 3 mg/kg/d groups (p<0.05). However, cardiac hypertrophy index is significantly lower in aliskiren 3 mg/kg/d treated compared to valsartan 10 mg/kg/d treated dTGR. Echocardiography shows a valsartan 1 mg/kg/d animal with concentric hypertrophy. Wall thickness is found to be about 3.4 mm with a normal left ventricular end-diastolic diameter. Treatment with aliskiren (3 mg/kg/d) or valsartan 10 mg/kg/d reduces wall thickness to about 2.2 mm and about 2.7 mm, respectively. Tissue Doppler measurements show an Ea/Aa ratio of about 0.68 in the valsartan 1 mg/kg/d group, while valsartan 10 mg/kg/d improves Ea/Aa quotient to about 1.0. Both high and low aliskiren doses increase Ea/Aa values to about 1.4 and about 1.5, respectively, demonstrating improved diastolic filling. Untreated dTGR at week 7, just prior to death, show already increases in LV thickness (about 3.5 mm), and have Ea to Aa ratio about 0.48, indicating diastolic dysfunction.

With RT-PCR, α-MHC mRNA and β-MHC expression are examined in the left ventricles. valsartan 10 mg/kg/d as well as both aliskiren treatments prevente the shift from α-MHC expression to the fetal β-MHC isoform. Aliskiren 3 mg/kg/d is most effective in this regard (p<0.05). LV ANP mRNA expression is reduced by both aliskiren treatments, compared to valsartan 1 mg/kg/d treated dTGR. Valsartan 10 mg/kg/d reduces the expression of this gene, but not to a significant degree.

The data show that the valsartan 1 mg/kg/d animals have severe left ventricular hypertrophy with marked diastolic dysfunction (diastolic heart failure). The LV hypertrophy is markedly ameliorated with valsartan 10 mg/kg/d and with both aliskiren doses. However, despite the regression of cardiac hypertrophy, diastolic dysfunction is still present in dTGR receiving high dose valsartan. Both aliskiren doses markedly improve diastolic dysfunction, with aliskiren 3 mg/kg/d resulting in the lowest wall thickness values and the best diastolic filling. In addition, the effects of aliskiren on gene expression of left ventricular α- and β-MHC isoforms, as well as atrial natriuretic peptide (ANP), are consistent with the cardioprotective effects that are observed with a renin inhibitor. The results demonstrate a molecular effect of renin inhibition on the myocardium.

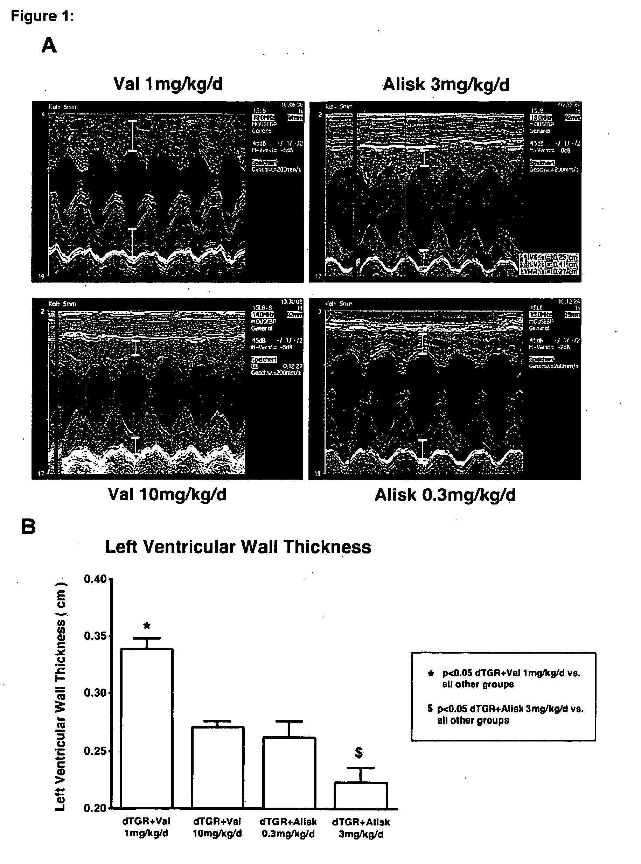

Figure 1: Shows M-mode echocardiography of LV septum and posterior wall of dTGR at 9 weeks of age. Panel A shows a valsartan (Val) 1 mg/kg/d rat with severe septal and posterior wall hypertrophy. Val 10 mg/kg/d reduces septal and posterior wall hypertrophy substantially. Aliskiren (Alisk) 0.3 mg/kg/d and Alisk 3 mg/kg/d also reduce left ventricular hypertrophy and the 3 mg/kg/d dose normalized LV dimensions. Panel B shows the quantification of the LV wall thickness. Results are mean±SEM. (n=10-14; * p<0.05 Val 1 mg/kg/d vs. other groups, $ Alisk 3 mg/kg/d vs. other groups).

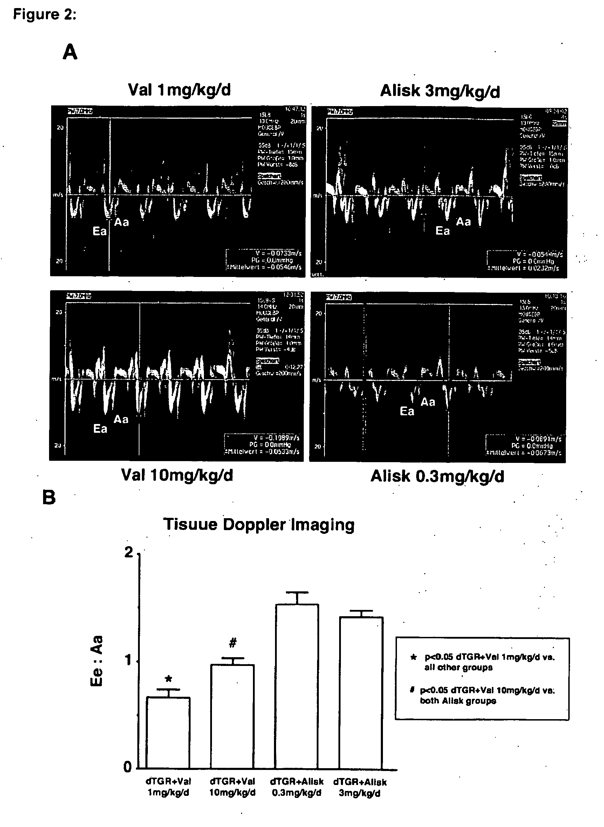

Figure 2: Shows Tissue Doppler assessment of diastolic filling in dTGR at 9 weeks of age: Ea wave (early diastolic filling) and the Aa wave (atrial contraction) of the same animals as are measured at the same time point. Val 1 mg/kg/d shows a deeper Aa than Ea wave, indicating a severe diastolic dysfunction (Ee/Ae=0.66). Val 10 mg/kg/d still showed similarly deep Ea and Aa waves indicating diastolic dysfunction (Ea/Aa 1.0). Alisk 0.3 mg/kg/d and Alisk 3 mg/kg/d show deeper Ea than Aa waves, indicating appropriate diastolic filling (Ea/Aa 1.5).

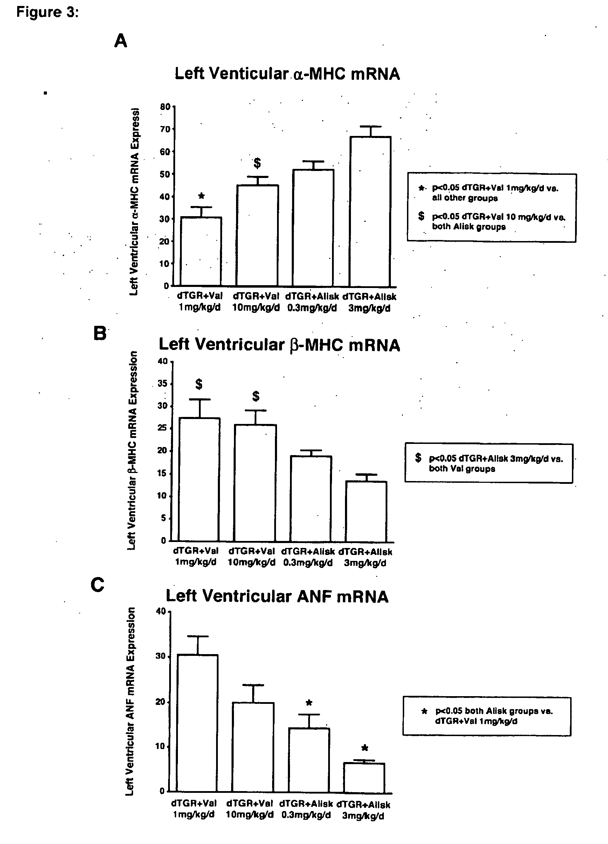

Figure 3: Shows the effects of treatment on markers of cardiac hypertrophy in 9 weeks old dTGR : Panel A shows a dose-related increase in α-MHC mRNA expression with the respective treatments, accompanied decreases in β-MHC (panel B) mRNA expression. Panel C shows a dose-related decrease in LV ANF mRNA expression with respective treatments. Results are mean±SEM (n=6 each).

Furthermore, it has been found that, a combination of a renin inhibitor, e.g., aliskiren, especially in the form of the hemi-fumarate salt thereof, and an ACE inhibitor, e.g., benazepril or enalapril, or an angiotensin II receptor blocker, e.g., valsartan, or in each case, a pharmaceutically acceptable salt thereof, achieves greater therapeutic effect than the administration of a renin inhibitor alone. Greater efficacy can also be documented as a prolonged duration of action. The duration of action can be monitored as either the time to return to baseline prior to the next dose or as the area under the curve (AUC).

Further benefits are that lower doses of the individual drugs to be combined according to the present invention can be used to reduce the dosage, e.g., that the dosages need not only often be smaller but are also applied less frequently, or can be used to diminish the incidence of side effects. The combined administration of a renin inhibitor, or a pharmaceutically acceptable salt thereof, and an ACE inhibitor, e.g., benazepril or enalapril, or an angiotensin II receptor blocker, e.g., valsartan, or in each case, a pharmaceutically acceptable salt thereof, results in a significant response in a greater percentage of treated patients, i.e., a greater responder rate results.

It can be shown that combination therapy with a renin inhibitor, e.g., aliskiren, especially in the form of the hemi-fumarate salt thereof, and an ACE inhibitor, e.g., benazepril or enalapril, or an angiotensin II receptor blocker, e.g., valsartan, or in each case, a pharmaceutically acceptable salt thereof, results in a more effective therapy for the prevention of, delay progression to overt to, or the treatment of diastolic dysfunction or diastolic heart failure. In particular, all the more surprising is the experimental finding that a combination of the present invention results in a beneficial, especially a synergistic, therapeutic effect but also in benefits resulting from combined treatment such as a surprising prolongation of efficacy.

The invention furthermore relates to the use of a renin inhibitor, e.g., aliskiren, alone or in combination with an ACE inhibitor, e.g., benazepril or enalapril, or an angiotensin II receptor blocker, e.g., valsartan, or in each case, a pharmaceutically acceptable salt thereof, for the manufacture of a medicament for the prevention of, delay progression to overt to, or the treatment of diastolic dysfunction or diastolic heart failure.

Accordingly, another embodiment of the present invention relates to the use of a renin inhibitor, e.g., aliskiren, alone or in combination with an ACE inhibitor, e.g., benazepril or enalapril, or an angiotensin II receptor blocker, e.g., valsartan, or in each case, a pharmaceutically acceptable salt thereof, for the manufacture of a medicament for the prevention of, delay progression to overt to, or the treatment of diastolic dysfunction or diastolic heart failure.

The above description fully discloses the invention including preferred embodiments thereof. Modifications and improvements of the embodiments specifically disclosed herein are within the scope of the following claims. Without further elaboration, it is believed that one skilled in the art can, using the preceding description, utilize the present invention to its fullest extent. Therefore, the Examples herein are to be construed as merely illustrative of certain aspects of the present invention and are not a limitation of the scope of the present invention in any way.

### Example 1:

### Composition of aliskiren 150 mg (free base) uncoated tablets in mg/unit.

| | **Roller compacted tablet** | **Dosage form 1** | **Dosage form 2** | **Dosage form 3** |
|---|---|---|---|---|
| **Component** | | | | |
| Aliskiren hemi-fumarate | 165.750 | 165.750 | 165.750 | 165.750 |
| Microcrystalline cellulose | 220.650 | 84.750 | 72.250 | 107.250 |
| Polyvinylpyrrolidon K 30 | - | - | 12.000 | 12.000 |
| Crospovidone | 84.000 | 45.000 | 44.000 | 48.200 |
| Aerosil 200 | 4.800 | 1.500 | 1.500 | 1.800 |
| Magnesium stearate | 4.800 | 3.000 | 4.500 | 5.000 |
| **Total weight** | **480.000** | **300.000** | **300.000** | **340.000** |

### Composition of aliskiren 150 mg (free base) uncoated tablets in % by weight.

| | **Roller compacted tablet** | **Dosage form 1** | **Dosage form 2** | **Dosage form 3** |
|---|---|---|---|---|
| **Component** | | | | |
| Aliskiren hemi-fumarate | 34.53 | 55.25 | 55.25 | 48.75 |
| Microcrystalline cellulose | 45.97 | 28.25 | 24.08 | 31.545 |
| Polyvinylpyrrolidon K 30 | - | - | 4 | 3.53 |
| Crospovidone | 17.5 | 15 | 14.67 | 14.175 |
| Aerosil 200 | 1 | 0.5 | 0.5 | 0.53 |
| Magnesium stearate | 1 | 1 | 1.5 | 1.47 |
| **Total %** | **100.00** | **100.00** | **100.00** | **100.00** |

### Composition of aliskiren 150 mg (free base) uncoated tablets in mg/unit (divided into inner/outer phase).

| | | **Roller compacted tablet** | **Dosage form 1** | **Dosage form 2** | **Dosage form 3** |
|---|---|---|---|---|---|
| | **Component** | | | | |
| **Inner Phase** | Aliskiren hemi-fumarate | 165.75 | 165.75 | 165.75 | 165.75 |
| | Microcrystalline cellulose | 220.65 | 84.75 | 72.25 | 90.25 |
| | Polyvinylpyrrolidon K 30 | - | - | 12.00 | 12.00 |
| | Crospovidone | 36.00 | - | - | 14.20 |
| | Aerosil 200 | - | - | - | - |
| | Magnesium stearate | 2.40 | - | - | - |
| **Outer phase** | Crospovidone | 48.00 | 45.00 | 44.00 | 34.00 |
| | Microcrystalline cellulose | - | - | - | 17.00 |
| | Aerosil 200 | 4.80 | 1.50 | 1.50 | 1.80 |
| | Magnesium stearate | 2.40 | 3.00 | 4.50 | 5.00 |
| | **Total weight** | **480.00** | **300.00** | **300.00** | **340.00** |

### Composition of aliskiren 150 mg (free base) uncoated tablets in % by weight (divided into inner/outer phase).

| | | **Roller compacted tablet** | **Dosage form 1** | **Dosage form 2** | **Dosage form 3** |
|---|---|---|---|---|---|
| | **Component** | | | | |
| **Inner Phase** | Aliskiren hemi-fumarate | 34.53 | 55.25 | 55.25 | 48.75 |
| | Microcrystalline cellulose | 45.97 | 28.25 | 24.08 | 26.545 |
| | Polyvinylpyrrolidon K 30 | - | - | 4 | 3.530 |
| | Crospovidone | 7.5 | - | - | 4.175 |
| | Aerosil 200 | - | - | - | - |
| | Magnesium stearate | 0.5 | - | - | - |
| **Outer phase** | Crospovidone | 10 | 15 | 14.67 | 10 |
| | Microcrystalline cellulose | - | - | - | 5 |
| | Aerosil 200 | 1 | 0.5 | 0.5 | 0.53 |
| | Magnesium stearate | 0.5 | 1 | 1.5 | 1.47 |
| | **Total %** | **100.00** | **100.00** | **100.00** | **100.00** |

### Example 2:

### Composition of aliskiren (dosage form 3) film-coated tablets in mg/unit.

| **Dosage form 3 / Strength** | **75 mg (free base)** | **150 mg (free base)** | **300 mg (free base)** |
|---|---|---|---|
| **Component** | | | |
| Aliskiren hemi-fumarate | 82.875 | 165.750 | 331.500 |
| Microcrystalline cellulose | 53.625 | 107.250 | 214.500 |
| Polyvinylpyrrolidon K 30 | 6.000 | 12.000 | 24.000 |
| Crospovidone | 24.100 | 48.200 | 96.400 |
| Aerosil 200 | 0.900 | 1.800 | 3.600 |
| Magnesium stearate | 2.500 | 5.000 | 10.000 |
| **Total tablet weight** | **170.000** | **340.000** | **680.000** |
| Opadry premix white | 9.946 | 16.711 | 23.9616 |
| Opadry premix red | 0.024 | 0.238 | 1.8382 |
| Opadry premix black | 0.030 | 0.051 | 0.2002 |
| **Total fim-coated tablet weight** | **180.000** | **357.000** | **706.000** |

The dosage forms 1, 2 and 3 may be prepared, e.g., as follows:
1) mixing the active ingredient and additives and granulating said components with a granulation liquid;
2) drying a resulting granulate;
3) mixing the dried granulate with outer phase excipients;
4) compressing a resulting mixture to form a solid oral dosage as a core tablet; and
5) optionally coating a resulting core tablet to give a film-coated tablet.

The granulation liquid can be ethanol, a mixture of ethanol and water, a mixture of ethanol, water and isopropanol, or a solution of polyvinylpyrrolidones (PVP) in the before mentioned mixtures. A preferred mixture of ethanol and water ranges from about 50/50 to about 99/1 (% w/w), most preferrably it is about 94/6 (% w/w). A preferred mixture of ethanol, water and isopropanol ranges from about 45/45/5 to about 98/1/1 (% w/w/w), most preferably from about 88.5/5.5/6.0 to about 91.5/4.5/4.0 (% w/w/w). A preferred concentration of PVP in the above named mixtures ranges from about 5 to about 30% by weight, preferably from about 15 to about 25%, more preferably from about 16 to about 22%.

Attention is drawn to the numerous known methods of granulating, drying and mixing employed in the art, e.g., spray granulation in a fluidized bed, wet granulation in a high-shear mixer, melt granulation, drying in a fluidized-bed dryer, mixing in a free-fall or tumble blender, compressing into tablets on a single-punch or rotary tablet press.

The manufacturing of the granulate can be performed on standard equipment suitable for organic granulation processes. The manufacturing of the final blend and the compression of tablets can also be performed on standard equipment.

For example, step (1) may be carried out by a high-shear granulator, e.g., Collette Gral; step (2) may be conducted in a fluid-bed dryer; step (3) may be carried out by a free-fall mixer (e.g. container blender, tumble blender); and step (4) may be carried out using a dry compression method, e.g., a rotary tablet press.

### Example 3 (film-coated tablets):

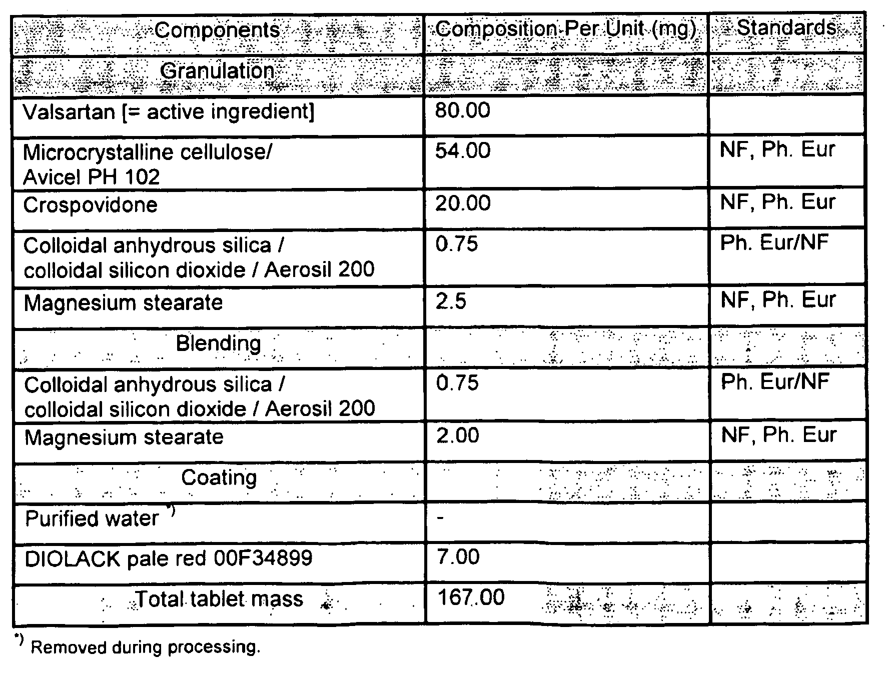

The film-coated tablets may be manufactured, e.g., as follows:

A mixture of valsartan, microcrystalline cellulose, crospovidone, part of the colloidal anhydrous silica/colloidal silicon dioxide/Aerosile 200, silicon dioxide and magnesium stearate is premixed in a diffusion mixer and then sieve through a screening mill. The resulting mixture is again pre-mixed in a diffusion mixer, compacted in a roller compactor and then sieve through a screening mill. To the resulting mixture, the rest of the colloidal anhydrous silica/colloidal silicon dioxide/Aerosile 200 are added and the final blend is made in a diffusion mixer. The whole mixture is compressed in a rotary tabletting machine and the tablets are coated with a film by using Diolack pale red in a perforated pan.

### Example 4 (film-coated tablets):

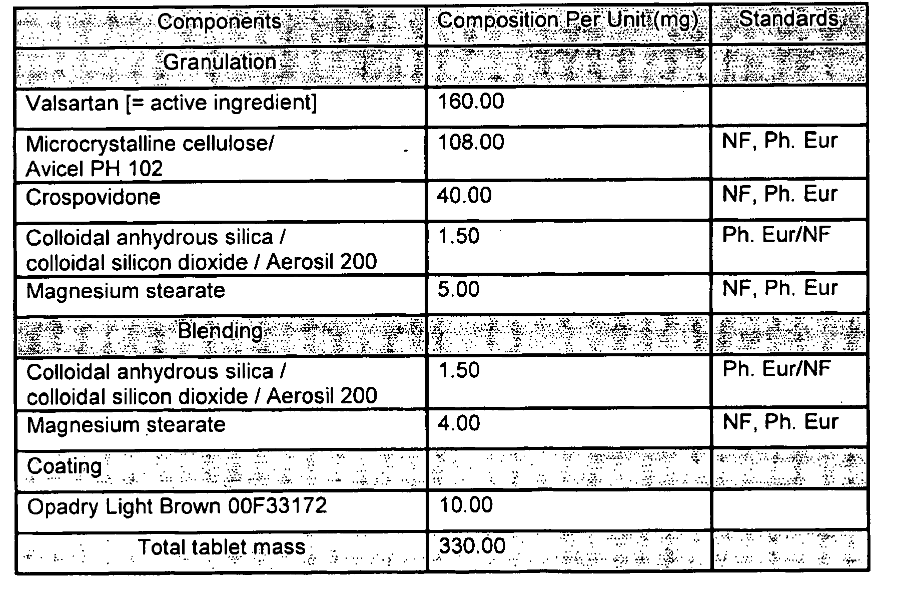

The film-coated tablets are manufactured, e.g., as described in Example 3.

### Example 5 (film-coated tablets):

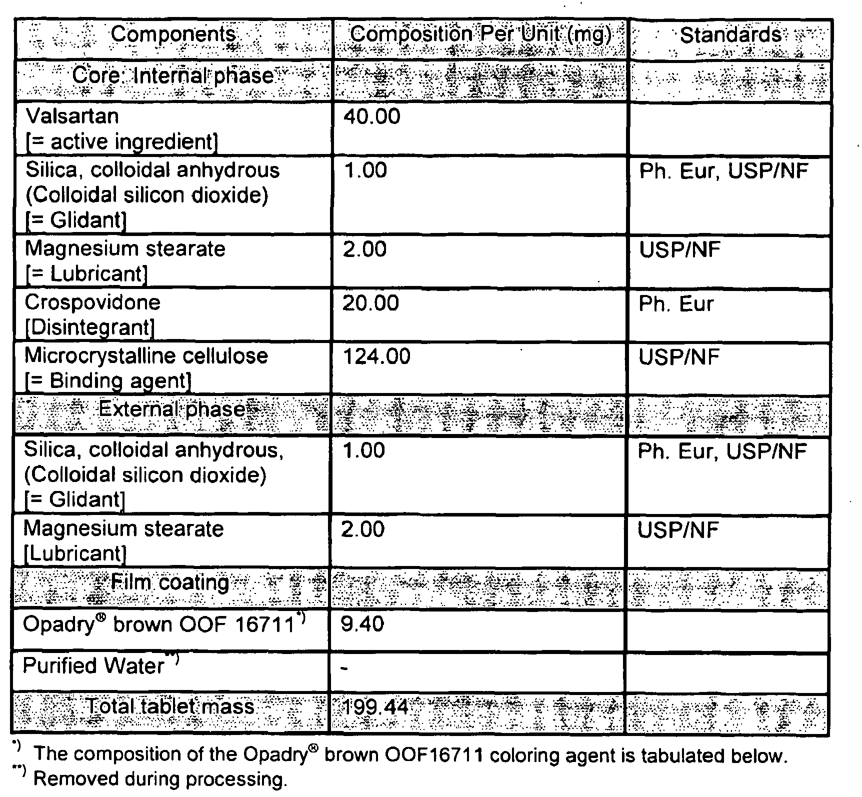

### Opadry^{®} Composition:

| Ingredient | Approximate % Composition |
|---|---|
| Iron oxide, black (C.I. No. 77499, E 172) | 0.50 |
| Iron oxide, brown (C.I. No. 77499, E 172 | 0.50 |
| Iron oxide, red (C.I. No. 77491, E 172) | 0.50 |
| Iron oxide, yellow (C.I. No. 77492, E 172) | 0.50 |
| Macrogolum (Ph. Eur) | 4.00 |
| Titanium dioxide (C.I. No. 77891, E 171) | 14.00 |
| Hypromellose (Ph. Eur) | 80.00 |

The film-coated tablets are manufactured, e.g., as described in Example 3.

### Example 6 (capsules):

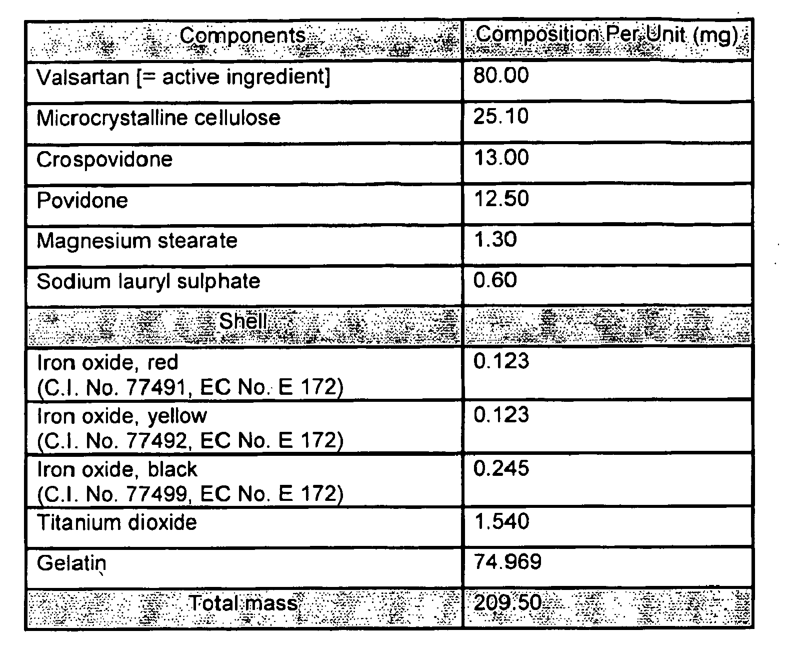

The capsules may be manufactured, e.g., as follows:

Granulation/Drying:
Valsartan and microcrystallin cellulose are spray-granulated in a fluidized bed granulator with a granulating solution consisting of povidone and sodium lauryl sulphate dissolved in purified water. The granulate obtained is dried in a fluidized bed dryer.

Milling/Blending:
The dried granulate is milled together with crospovidone and magnesium stearate. The mass is then blended in a conical srew type mixer for approximately 10 minutes.

Encapsulation:
The empty hard gelatin capsules are filled with the blended bulk granules under controlled temperature and humidity conditions. The filed capsules are dedusted, visually inspected, weightchecked and quarantined until by Quality assurance department.

### Example 7 (capsules):

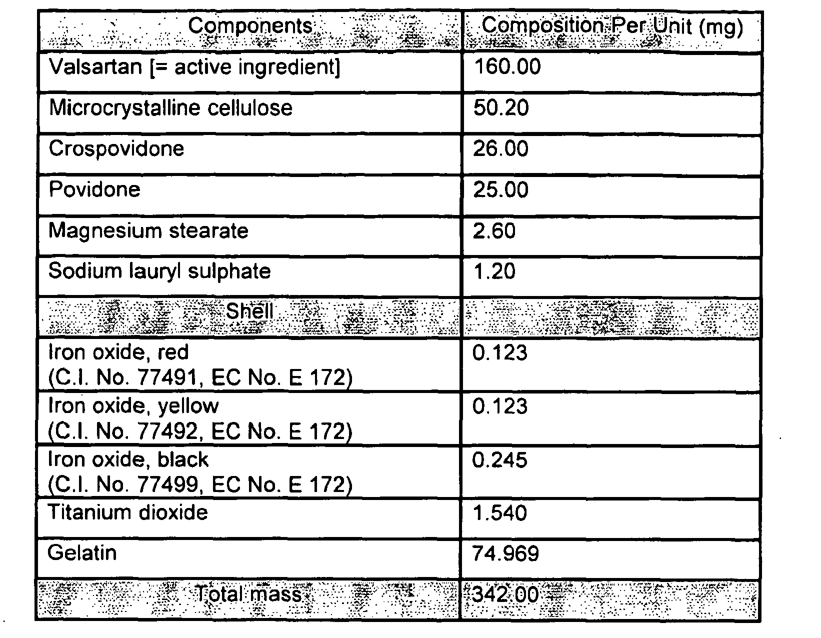

The capsules are manufactured, e.g., as described in Example 6.

### Example 8 (hard gelatine capsules):

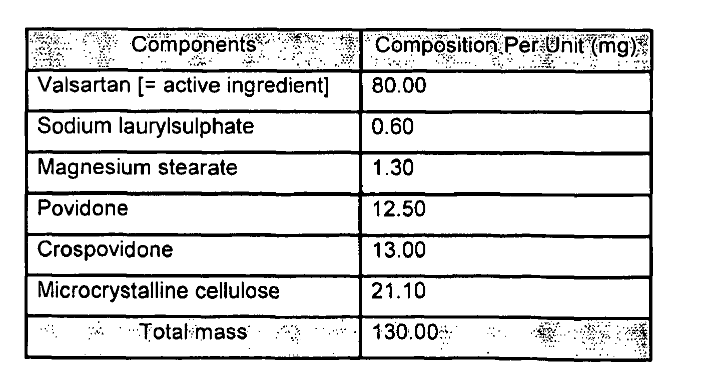

### Example 9 (hard gelatin capsules):

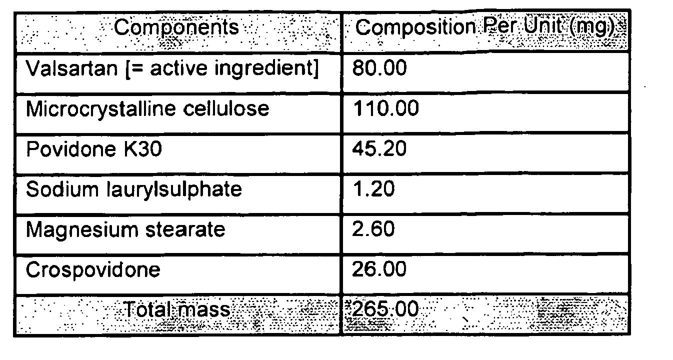

Components (1) and (2) are granulated with a solution of components (3) and (4) in water. The components (5) and (6) are added to the dry granulate and the mixture is filled into size 1 hard gelatin capsules.

All publications and patents mentioned herein are incorporate by reference in their entirety as if set forth in full herein.

## Claims

1. A pharmaceutical composition comprising a renin inhibitor for the prevention of, delay progression to overt to, or the treatment of diastolic dysfunction or diastolic heart failure.

2. The composition according to claim 1 , wherein a renin inhibitor is selected from the group consisting of RO 66-1132, RO 66-1168 and a compound of formula (III) 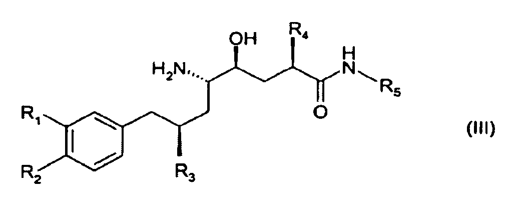 wherein R₁ is halogen,C₁₋₆halogenalkyl, C₁₋₆alkoxy-C₁₋₆alkyloxy or C₁₋₆alkoxy-C₁₋₆alkyl; R₂ is halogen, C₁₋₄alkyl or C₁₋₄alkoxy; R₃ and R₄ are independently branched C₃₋₆alkyl; and R₅ is cycloalkyl, C₁₋₆alkyl, C₁₋₆hydroxyalkyl, C₁₋₆alkoxy-C₁₋₆alkyl, C₁₋₆alkanoyloxy-C₁₋₆alkyl, C₁₋₆aminoalkyl, C₁₋₆alkylamino-C₁₋₆alkyl, C₁₋₆dialkylamino-C₁₋₆alkyl, C₁₋₆alkanoylamino-C₁₋₆alkyl, HO(O)C-C₁₋₆alkyl, C₁₋₆alkyl-O-(O)C-C₁₋₆alkyl, H₂N-C(O)-C₁₋₆alkyl, C₁₋₆ alkyl-HN-C(O)-C₁₋₆alkyl or (C₁₋₆alkyl)₂N-C(O)-C₁₋₆alkyl; or in each case a pharmaceutically acceptable salt thereof.

3. The composition according to claim 2, wherein a renin inhibitor is a compound of formula (III) having the formula 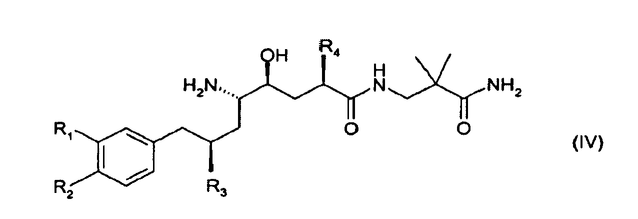 wherein R₁ is 3-methoxypropyloxy; R₂ is methoxy; and R₃ and R₄ are isopropyl; or a pharmaceutically acceptable salt thereof.

4. The composition according to claim 3, wherein the compound of formula (IV) is in the form of the hemi-fumarate salt thereof.

5. Use of a renin inhibitor for the manufacture of a medicament for the prevention of, delay progression to overt to, or the treatment of diastolic dysfunction or diastolic heart failure.

6. Use according to claim 5, wherein a renin inhibitor is selected from a group consisting of RO 66-1132, RO 66-1168 and a compound of the formula 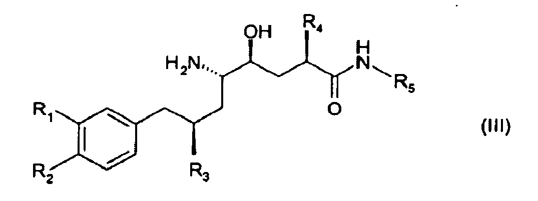 wherein R₁ is halogen, C₁₋₆halogenalkyl, C₁₋₆alkoxy-C₁₋₆alkyloxy or C₁₋₆alkoxy-C₁₋₆alkyl; R₂ is halogen, C₁₋₄alkyl or C₁₋₄alkoxy; R₃ and R₄ are independently branched C₃₋₆alkyl; and R₅ is cycloalkyl, C₁₋₆alkyl, C₁₋₆hydroxyalkyl, C₁₋₆alkoxy-C₁₋₆alkyl, C₁₋₆alkanoyloxy-C₁₋₆alkyl, C₁₋₆aminoalkyl, C₁₋₆alkylamino-C₁₋₆alkyl, C₁₋₆dialkylamino-C₁₋₆alkyl, C₁₋₆alkanoylamino-C₁₋₆alkyl, HO(O)C-C₁₋₆alkyl, C₁₋₆alkyl-O-(O)C-C₁₋₆alkyl, H₂N-C(O)-C₁₋₆alkyl, C₁₋₆alkyl-HN-C(O)-C₁₋₆alkyl or (C₁₋₆alkyl)₂N-C(O)-C₁₋₆alkyl; or in each case a pharmaceutically acceptable salt thereof.

7. Use according to claim 6, wherein a compound of formula (III) has the formula 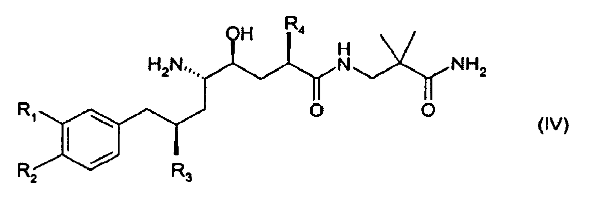 wherein R₁ is 3-methoxypropyloxy; R₂ is methoxy; and R₃ and R₄ are isopropyl; or a pharmaceutically acceptable salt thereof.

8. Use according to claim 7, wherein the compound of formula (IV) is in the form of the hemi-fumarate salt thereof.
